Europäisches Patentamt

European Patent Office

Office européen des brevets

(19)

(11) Publication number: 0 295 827
A2

# EUROPEAN PATENT APPLICATION

(21) Application number: 88305279.7

(51) Int. Cl.⁴: A61K 31/59

(22) Date of filing: 09.06.88

(30) Priority: 10.06.87 JP 144328/87

(43) Date of publication of application:
21.12.88 Bulletin 88/51

(84) Designated Contracting States:
DE FR GB IT

(71) Applicant: KUREHA KAGAKU KOGYO
KABUSHIKI KAISHA
1-9-11, Nihonbashi, Horidome-cho
Chuo-ku Tokyo 103(JP)

(72) Inventor: Yamato, Hideyuki
5-24-21 Takenotsuka
Adachi-ku Tokyo(JP)
Inventor: Maeda, Yuji
2-609 Minami-Nagareyama-Ichibangai
2013 Nagareyama Nagareyama-shi
Chiba-ken(JP)
Inventor: Hirai, Toru
5-31-1 Kasumigaseki-Kita
Kawagoe-shi Saitama-ken(JP)
Inventor: Ikuzawa, Masanori
1-25-20 Wakaba-cho
Tachikawa-shi Tokyo(JP)
Inventor: Togawa, Masanori
604-8 Gakuen-Higashi-machi
Kodaira-shi Tokyo(JP)
Inventor: Hirose, Fumio
22 Kureha-Kagaku-Ichigaya-So 8
Ichigayadai-machi
Shinjuku-ku Tokyo(JP)
Inventor: Nemoto, Michiichiro
740-1-1115 Oaza Miyawada Fujishiro-machi
Kita-Soma-gun Ibaraki-ken(JP)
Inventor: Kato, Tadaaki
K-407, 3-27 Nakadai
Itabashi-ku Tokyo(JP)
Inventor: Yoshikumi, Chikao
2-19-46 Higashi
Kunitachi-shi Tokyo(JP)

(74) Representative: Woods, Geoffrey Corlett et al
J.A. KEMP & CO. 14 South Square Gray's Inn
London WC1R 5EU(GB)

(54) Use of 24,25-dihydroxy-cholecalciferol in mending bone fractures.

(57) 24,25-Dihydroxycholecalciferol is·administered orally or rectally to accelerate the mending of a bone fracture.

# USE OF 24,25-DIHYDROXYCHOLECALCIFEROL IN MENDING BONE FRACTURES

The present invention relates to the use of 24,25-dihydroxycholecalciferol, hereinafter referred to as "24,25-$(OH)_2$-$D_3$".

Bone fractures occur in persons of all ages, from newborn babies to old people. Bones have various shapes, and are divided broadly into long bones, flat bones and short bones. The surface of each bone is covered with dense bone. The interior of each bone is spongy and cancellous. Fracture means the phenomenon where the continuity of such a bone structure is partially or completely broken, the former being called an incomplete fracture and the latter a complete fracture.

Recently, not to mention an increase in fractures in children, there has been an increase in fractures in old people due to the progress of an advanced-aged society. This has become an important problem. It is said that the time required to cure a fracture in an adult is prolonged in proportion to the age of the adult and that it is often the case with advanced-aged persons that fractures are not completely cured.

At present, fractures are treated, as a rule, by long-known methods such as manipulative reduction and immobilization, extension and plaster fixation. Operations are performed only in a few cases and treatment of fractures fundamentally depends on natural cure.

In this state, development of a medicine for accelerating the cure of a fracture, which is highly safe, easy to administer, and capable of repetitive administration has been expected.

It has already been described in Japanese Patent Laid-Open (KOKAI) No. 61-222452 (1986) that 24,25-$(OH)_2$-$D_3$ is effective to fractures. In the methods disclosed in the above-described specification, however, 24,25-$(OH)_2$-$D_3$ must be administered by local injection or direct administration into the affected part, so that the patient suffers a pain and, in addition, must receive treatment in hospital or go to hospital regularly.

As to an excipient, the safety thereof is indistinct in the respect of antigenicity, for example, and adaptation is difficult in some states of the fractured part.

It is also clear that repetitive administration is difficult in these methods. Thus, these methods are actually very difficult to adopt.

As a result of further studies of the effect of 24,25-$(OH)_2$-$D_3$ on the treatment of a fracture including the route of administration, the present inventors have found that the cure of a fracture is remarkably accelerated by the oral administration and has made clear that 24,25-$(OH)_2$-$D_3$ is adaptable to the treatment of a fracture as an oral medicine.

In an aspect of the present invention, there is provided a use of 24,25-dihydroxycholecalciferol for manufacturing an accelerator for curing a fracture.

24,25-$(OH)_2$-$D_3$ may be any of 24R,25-$(OH)_2$-$D_3$, 24S,25$(OH)_2$-$D_3$ and a mixture thereof. Among these, 24R,25-$(OH)_2$-$D_3$ is particularly preferred. The fracture cure accelerating agent used in the present invention contains 24,25-$(OH)_2$-$D_3$ as the active ingredient and is used in the various forms which will be described below. The fracture cure accelerating agent may be administered either orally or through the rectum, but oral administration is preferable.

A medicine containing 24,25-$(OH)_2$-$D_3$ as the active ingredient is administered in the form of a tablet, powder, granules, suppository, capsule, alcoholic solution, oily solution, aqueous suspension or the like. As the oily solvent, triglyceride esters of middle fatty acids, corn oil, cottonseed oil, peanut oil, fish liver oil, oily esters and the like are usable. Cocoa butter and glycerin are also preferable. Lactose, starch, talk, magnesium stearate, sorbic acid, salts of sorbic acid, titanium white, gelatin, sugars, derivatives of sugars, alcohols, physiologic saline, surfactants, antioxidants, antiseptics, etc. are usable together with 24,25-$(OH)_2$-$D_3$ as another ingredient.

The preferable amount of 24,25-$(OH)_2$-$D_3$ contained in a unit dosage form is 0.00002 to 4 wt%, more preferably 0.0002 to 1 wt%. The dosage of 24,25-$(OH)_2$-$D_3$ for adults is 0.1 to 100,000 μg, preferably 0.5 to 10,000 μg a day.

The acute toxicity of 24,25-$(OH)_2$-$D_3$ was examined in the following manner.

Acute toxicity:

24,25-$(OH)_2$-$D_3$ in ethanol was dissolved in a triglyceride ester of a middle fatty acid so as to have an ethanol concentration of 2 %. The thus-prepared solution was orally administered to 10 ICR male mice (weight: 25 ± 3g). The dosage of 24,25-$(OH)_2$-$D_3$ was 100 mg/kg. For two weeks after the administration, the toxic symptoms of the mice were observed, but all of the ten mice existed without any abnormality. When they were subjected to the biochemical blood test, anatomical examination and pathological histology after they were killed, there was no difference between the treated mice and a controlled group of mice to which only a triglyceride ester of a middle fatty acid containing 2% ethanol had been administered. Since the $LD_{50}$ value of the oral administration of 24,25-$(OH)_2$-$D_3$ was not less than 100 mg/kg, 24,25-$(OH)_2$-$D_3$ can be said to be very safe.

The advantages of the present invention will be explained more precisely with reference to the following examples. The absolute configuration at the position 24 of 24R,25-$(OH)_2$-$D_3$ used in the examples were confirmed according to the method on pp. 2203 to 2206 of Tetrahedron Letters No. 26 (1975).

Example 1

Five mg of 24R,25-$(OH)_2$-$D_3$ was dissolved in 1 kg of a triglyceride ester of a middle fatty acid which had been irradiated with a 400 W high-pressure mercury lamp under argon bubbling to remove impure reactive peroxides therefrom. A soft capsuled medicine containing 0.5 $\mu$g of 24R,25-$(OH)_2$-$D_3$ was prepared by dissolving the following materials for capsule wall under heating into the thus-obtained 24R,25-$(OH)_2$-$D_3$ solution and by using a soft capsule manufacturing machine.

Materials for capsule wall

Gelatin      10 parts by weight
Glycerin      2 parts by weight
Antiseptic      0.05 part by weight
(ethylparaben)
Titanium white      0.2 part by weight
Water      0.2 part by weight (final)

Also, soft capsuled medicines containing 1$\mu$g, 2 $\mu$g, 5 $\mu$g and 10 $\mu$g, respectively, of 24R,25-$(OH)_2$-$D_3$ in one capsule were prepared in the same way.

Example 2

24R,25-$(OH)_2$-$D_3$ was dissolved in 1% ethanol containing Panacate 810 to a predetermined concentration and the resultant solution was forcibly and orally administered to 5-week-old male or female ICR mice for 30 days in succession so that the does might become to 10 $\mu$g/kg/day, 100 $\mu$g/kg/day and 1,000 $\mu$g/kg/day, respectively. The thus treated mice were compared with a group of mice to which only the solvent had been administered in the following test items. The results are shown in the following tables. According to the growth curve of the measured weights, no significant difference between the groups was observed.

The following organs were fixed by 10% formalin, stained with hematoxylin-eosin and subjected to pathological inspection. No particular abnormality was observed:

brain, heart, lung, liver, kidney, adrenal gland, spleen, pancreas, thyroid gland, hypophysis, thymus, mesentric lymphgland, testis, ovary, uterus, stomach, small intestines (jejunum, ilium and duodenum), large intestines (colon and caecum), eyeball, submandibular gland, urinary bladder, dorsal skin, muscle, sternum, medulla of sternum, femur and medulla of femur.

## Table 1-1

### General blood test

| Gender | Group (10 mice) | Dosage (µg/kg·day) | Number of erythrocyte | Number of leucocyte | Amount of hemoglobin | Homatocrit |
|--------|-----------------|--------------------|-----------------------|---------------------|----------------------|------------|
| male | I | 10 | → | → | → | → |
| | II | 100 | → | → | → | → |
| | III | 1000 | → | → | → | → |
| female | IV | 10 | → | → | → | → |
| | V | 100 | → | → | → | → |
| | VI | 1000 | → | → | → | → |

In comparison with the control group:

↓ : Decreased

→ : No change

↑ : Increased

EP 0 295 827 A2

EP 0 295 827 A2

## Table 1-2

### Biochemical serum test

| Dosage (μg/kg·day) | Gender | GOT | GPT | LDH | Ca | I-P | ALP | T-P | A/G | Alb | T-Bil | Glu | T-CHO | BUN |
|---|---|---|---|---|---|---|---|---|---|---|---|---|---|---|
| 10 | male | → | → | → | → | → | → | → | → | → | → | → | → | → |
| 100 | male | → | → | → | → | → | → | → | → | → | → | → | → | → |
| 1000 | male | → | → | ↓ | → | → | → | → | → | → | ↓ | ↓ | → | ↓ |
| 10 | female | → | → | → | → | → | → | → | → | → | → | → | → | → |
| 100 | female | → | → | → | → | → | → | → | → | → | → | → | → | → |
| 1000 | female | → | → | → | → | → | → | → | → | → | → | → | → | → |

In comparison with the control group:

↓ : Decreased

→ : No change

↑ : Increased

## Table 1-3

### Urine analysis

| Dosage (μg/kg) | Gender | pH | Sugar | Protein | Occult blood | Ketone body | Urobilinogen |
|---|---|---|---|---|---|---|---|
| 10 | | → | → | → | → | → | → |
| 100 | male | → | → | → | → | → | → |
| 1000 | | → | → | → | → | → | → |
| 10 | | → | → | → | → | → | → |
| 100 | female | → | → | → | → | → | → |
| 1000 | | → | → | → | → | → | → |

In comparison with the control group:

↓ : Decreased

→ : No change

↑ : Increased

EP 0 295 827 A2

## Table 1-4

## Weight of organs

| Dosage (μg/ kg·day) | Gender | Brain | Hypo-physis | Heart | Lung | Liver | Spleen | Kidney | Adrenal gland | Thymus | Testis | Ovary | Uterus |
|---|---|---|---|---|---|---|---|---|---|---|---|---|---|
| 10 | male | → | → | → | → | → | → | → | → | → | → | | |
| 100 | | → | → | → | → | → | → | → | → | → | → | | |
| 1000 | | → | → | → | → | → | → | → | → | → | → | | |
| 10 | gemale | → | → | → | → | → | → | → | → | → | | → | → |
| 100 | | → | → | → | → | → | → | → | → | → | | → | → |
| 1000 | | → | → | → | → | → | → | → | → | → | | → | → |

In comparison with the control group:

↓ : Decreased

→ : No change

↑ : Increased

EP 0 295 827 A2

Example 3

The right fibulae of mature house rabbits (Japanese white house rabbits having a weight of about 3 kg) were broken at the central portions under anesthetic with a dental hand drill. Each of the house rabbits was kept in a separate cage and was allowed to take an ordinary solid food (CR-1, produced by Nippon Clare Corp.) and city water freely. $24R,25\text{-}(OH)_2\text{-}D_3$ and $24S,25\text{-}(OH)_2\text{-}D_3$ were dispersed in an aqueous 0.5% CMC solution and orally administered to the house rabbits through a rubber catheter. The experimental groups were as follows:

| No. | Group | Medicine and dosage | Number of rabbits |
|---|---|---|---|
| 1 | Control group of rabbits suffering fracture | Aqueous 0.5% CMC solution Oral administration | 6 |
| 2 | Group of rabbits suffering fracture | $24R,25\text{-}(OH)_2\text{-}D_3$ 100 µg/kg | 6 |
| 3 | Group of rabbits suffering fracture | $24S,25\text{-}(OH)_2\text{-}D_3$ 100 µg/kg | 6 |
| 4 | Group of normal rabbits | Aqueous 0.5% CMC solution Oral administration | 6 |

The respective medicines or aqueous 0.5% CMC solution was administered to these house rabbits for 3 weeks or 6 weeks after the bones had been broken. Thereafter, the house rabbits were killed and the fibulae were taken out for X-ray inspection and a strength test (bending test by DDV-VMF produced by Toyo Baldwin Co., Ltd.). When the house rabbits were inspected through X rays 3 weeks after the bones had been broken, the broken state was observed in the control group (Group 1) of rabbits suffering a fracture, but it was confirmed that in the group of rabbits to which $24R,25\text{-}(OH)_2\text{-}D_3$ had been administered, the cure of the fracture was accelerated.

The results of the strength test are shown in Table 2.

## Table 2

(average value $\pm$ standard deviation)

| Group | Bending strength ( N/mm ) | |
|---|---|---|
| | 3 weeks after | 6 weeks after |
| 1 | anmeasurable | ·77 $\pm$ 23 |
| 2 | 63 $\pm$ 33 | 103 $\pm$ 15* |
| 3 | 15 | 81 $\pm$ 37 |
| 4 | 98 $\pm$ 20 | 98 $\pm$ 20 |

*: $p < 0.05$ (average value $\pm$ standard deviation)

From Table 2, it is obvious that $24,25\text{-}(OH)_2\text{-}D_3$, in particular, $24R,25\text{-}(OH)_2\text{-}D_3$ is excellent in the accelerating effect on the cure of a fracture.

## Claims

1. Use of 24,25-dihydroxycholecalciferol in the manufacture of a pharmaceutical composition for oral or rectal administration to accelerate the mending of a bone fracture.

2. Use according to claim 1, wherein the said 24,25-dihydroxycholecalciferol is 24R,25-dihydroxycholecalciferol.

3. Use according to claim 1 or 2, wherein an orally administrable composition is manufactured in the form of tablets, a powder, granules, capsules, an alcohol solution, an oily solution or an aqueous suspension.

4. Use according to claim 1 or 2, wherein a suppository is manufactured.